# NEW EUROPEAN PATENT SPECIFICATION

(11) **EP 3 010 396 B2**
(45) Date of publication and mention of the opposition decision: **04.06.2025**
(45) Mention of the grant of the patent: 01.01.2020
(21) Application number: 14726183.8
(22) Date of filing: 08.05.2014
(51) Int. Cl.: A61B 5/00, A61M 16/00

(54) **RESPIRATORY THERAPY APPARATUS**
ATEMTHERAPIEVORRICHTUNG
APPAREIL DE THÉRAPIE RESPIRATOIRE

(30) Priority: 18.06.2013 GB 201310824
(43) Date of publication of application: 27.04.2016
(73) Proprietor: Smiths Medical International Limited, Ashford, Kent TN25 4BF (GB)
(72) Inventor: BENNETT, Paul James Leslie, Marston Moretaine Bedfordshire MK43 0AQ (GB)
(74) Representative: Charrier Rapp & Liebau
(86) International application number: PCT/GB2014/000177
(87) International publication number: WO 2014/202923

(56) References cited:
- EP-A1- 1 908 489
- WO-A2-2012/026963
- WO-A2-2012/038903
- WO-A2-2012/058727
- US-A1- 2004 187 871
- US-A1- 2011 125 063
- US-B1- 6 581 598
- BRIGITTE BUOZIN: "La Kiné sans accroc ou accro à la Kiné", MUCOVISCIDOSE, no. 117, February 2007 (2007-02-01), pages 30 - 31, XP055768750
- Allan Dunphy Acapella Mucous Clearance Device" Youtube.28 October 2012. 2012-10-28)

## Description

This invention relates to respiratory therapy apparatus of the kind including a vibratory PEP therapy device including a valve that is opened and closed by breathing through the device such as to produce an oscillating resistance to breathing through the device, the apparatus includes a sensor responsive to acoustic pressure waves transmitted through air caused by movement of the valve, the sensor is arranged to provide a signal indicative of use of the device, and the sensor is separate from the device but placed close to it to receive acoustic pressure waves transmitted from the device through air to the sensor.

The invention is also concerned with methods of evaluating patient use of respiratory therapy apparatus.

Positive expiratory pressure (PEP) apparatus, that is, apparatus that presents a resistance to expiration through the device, are now widely used to help treat patients suffering from a range of respiratory impairments, such as chronic obstructive pulmonary disease, bronchitis, cystic fibrosis and atelectasis. More recently, such apparatus that provide an alternating resistance to flow have been found to be particularly effective. One example of such apparatus is sold under the trade mark Acapella (a registered trade mark of Smiths Medical) by Smiths Medical and is described in US6581598, US6776159, US7059324 and US7699054. Other vibratory respiratory therapy apparatus is available, such as "Quake" manufactured by Thayer, "AeroPEP" manufactured by Monaghan, "TheraPEP" manufactured by Smiths Medical and "IPV Percussionator" manufactured by Percussionaire Corp. Alternative apparatus such as "CoughAssist" manufactured by Philips is also available. Respiratory therapy apparatus can instead provide an alternating resistance to flow during inhalation. An further example of a respiratory therapy apparatus including a valve is disclosed in EP 1 908 489.

To be effective these apparatus must be used regularly at prescribed intervals. In the case of chronic diseases, the patient needs to use the apparatus daily for the rest of his life in order to maintain a continuous relief.

Although these apparatus can be very effective, users often neglect to use the apparatus regularly at the prescribed frequency. It is very difficult to maintain a record of use of the apparatus, especially when the patient is using it at home. The clinician often does not know whether deterioration in a patient's condition is because he has failed to use the apparatus as prescribed or whether other factors are the cause.

It is an object of the present invention to provide alternative respiratory therapy apparatus.

According to the present invention there is provided a respiratory therapy apparatus of the above-specified kind, characterised in that the valve includes a valve element on a rocker arm that opens and closes an opening during exhalation through the apparatus, the oscillating movement of the rocker arm producing an audible sound being transmitted through the surrounding air as the acoustic pressure waves, the sensor is arranged to provide a signal indicative of the frequency of oscillation, the sensor is contained in a unit including a display on which is represented the frequency of oscillation detected by the sensor.

The sensor may be arranged to provide a signal indicative of one or more of the following: when the device is used and the duration of use. The unit may be a mobile phone and the sensor may be the microphone of the phone, the phone being programmed to respond to the sound made by the apparatus during use.

Apparatus including a vibratory PEP device will now be described, by way of example, with reference to the accompanying drawings, in which:
- Figure 1: is an exploded view of the apparatus;
- Figure 2: illustrates the sound output produced by a single breath;
- Figure 3: illustrates the apparatus in use;
- Figure 4A: is a front elevation view of one form of a dedicated sensor; and
- Figure 4B: is a side elevation view of the sensor shown in Figure 4A.

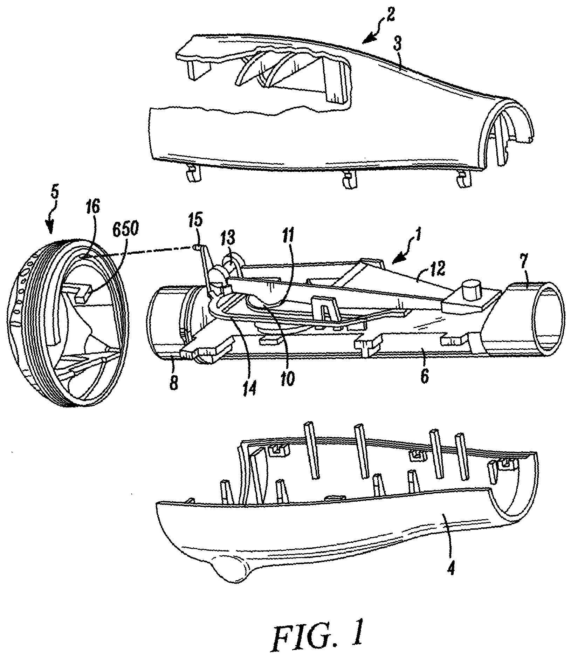

-With reference first to Figure 1, the device 100 comprises a rocker assembly 1 contained within an outer housing 2 provided by an upper part 3 and a lower part 4 of substantially semicylindrical shape. The device is completed by an adjustable dial 5 of circular section. The rocker assembly 1 includes an air flow tube 6 with a breathing inlet 7 at one end and an inspiratory inlet 8 at the opposite end including a one-way valve (not shown) that allows air to flow into the air flow tube 6 but prevents air flowing out through the inspiratory inlet. The air flow tube 6 has an outlet opening 10 with a non-linear profile that is opened and closed by a conical valve element 11 mounted on a rocker arm 12 pivoted midway along its length about a transverse axis. The air flow tube 6 and housing 2 provide a structure with which the rocker arm 12 is mounted. At its far end, remote from the breathing inlet 7, the rocker arm 12 carries an iron pin 13 that interacts with the magnetic field produced by a permanent magnet (not visible) mounted on an adjustable support frame 14. The magnet arrangement is such that, when the patient is not breathing through the device, the far end of the rocker arm 12 is held down such that its valve element 11 is also held down in sealing engagement with the outlet opening 10. A cam follower projection 15 at one end of the support frame 14 locates in a cam slot 16 in the dial 5 such that, by rotating the dial, the support frame 14, with its magnet, can be moved up or down to alter the strength of the magnetic field interacting with the iron pin 13. The dial 5 enables the frequency of operation and the resistance to flow of air through the device to be adjusted for maximum therapeutic benefit to the user.

When the patient inhales through the breathing inlet 7 air is drawn through the inspiratory inlet 8 and along the air flow tube 6 to the breathing inlet. When the patient exhales, the one-way valve in the inspiratory inlet 8 closes, preventing any air flowing out along this path. Instead, the expiratory pressure is applied to the underside of the valve element 11 on the rocker arm 12 causing it to be lifted up out of the opening 10 against the magnetic attraction, thereby allowing air to flow out to atmosphere. The opening 10 has a non-linear profile, which causes the effective discharge area to increase as the far end of the rocker arm 12 lifts, thereby allowing the arm to fall back down and close the opening. As long as the user keeps applying sufficient expiratory pressure, the rocker arm 12 will rise and fall repeatedly as the opening 10 is opened and closed, causing a vibratory, alternating or oscillating resistance to expiratory breath flow through the device. Further information about the construction and operation of the device can be found in US6581598, the contents of which are hereby incorporated into the present application.

As so far described, the apparatus is conventional.

The apparatus of the present invention includes the device 100 described above and sensor means 20 responsive to pressure waves transmitted through air and caused by use of the device.

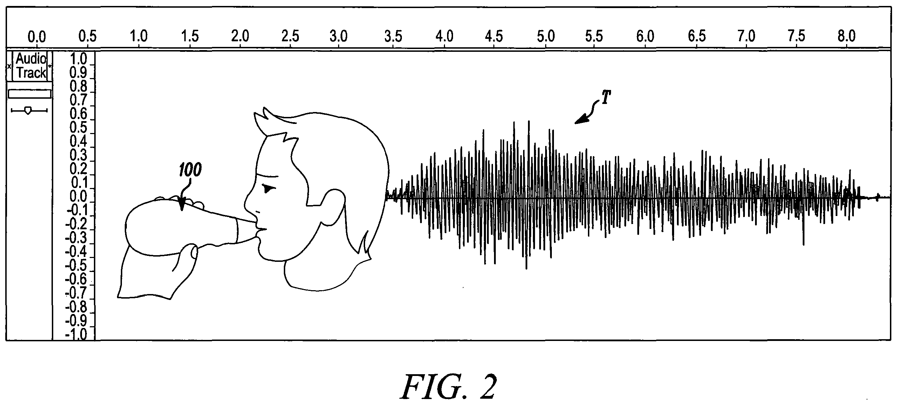

Figure 2 illustrates the patient exhaling through the device 100 to produce the desired therapy effect. The oscillating movement of the rocker arm 12 produces an audible sound that is transmitted through the surrounding air as pressure waves. This is represented by the trace "T" of a single expiration breath shown at the right of the Figure. It can be seen that the sensed sound takes the form of rapidly alternating positive and negative peaks at a frequency dependent on the frequency of rocking of the rocker arm 12 and with a mean amplitude that rises to a maximum towards the start of the breath and then gradually tails off towards the end of the breath.

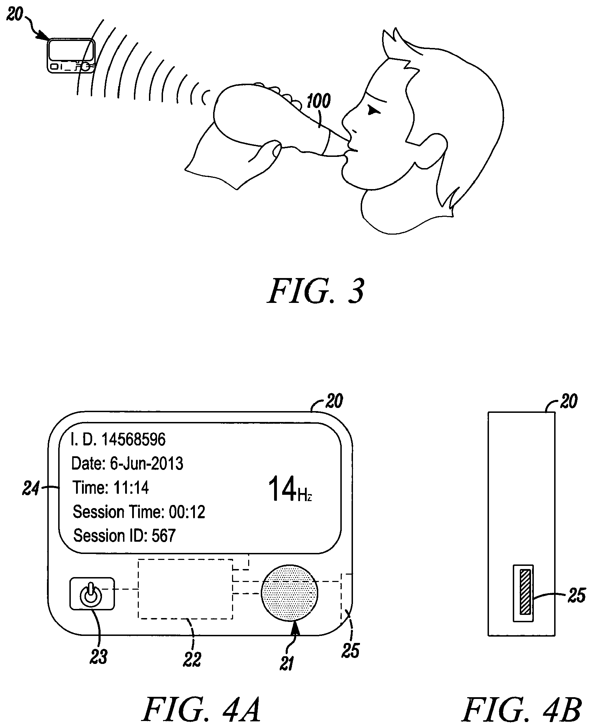

Figures 3 and 4 show the device 100 and sensor means in the form of a stand-alone acoustic sensor unit 20 that is separate from the device but, in use, is placed close to it. The sensor unit 20 includes a microphone 21 connected to a processing and memory unit 22, which is also connected to an on/off button 23 and a display screen 24. The microphone 21 is preferably responsive to sound in the audible hearing range. The unit 20 also has a data interface, such as the USB port 25 shown, or a wireless interface, such as a radio frequency Bluetooth or an infra-red interface. The unit 20 may also include input means by which information can be entered to the sensor unit, or this could be carried out via the data interface 25 from a remote computer or the like.

In use, the sensor unit 20 is placed close to the device 100, within the audible range of the microphone 21, but is not in direct contact with the device. The sensor unit 20 is turned on using the button 23 and the display 24 shows a representation of the user's identification, such as in the form of a unique number, the date and present time. When the user starts the therapy session the device 100 starts to emit sound waves that are picked up by the microphone 21 and appropriately processed by the processing unit 22. The processing unit 22 can measure various parameters, such as the duration of each exhalation, the number of exhalations in each session, the amplitude and amplitude profile of each sensed exhalation and the oscillation frequency during exhalation. As illustrated in Figure 4A, the screen 24 provides the user with a representation of the session time and the frequency of oscillation, which has been found to be particularly important to users in achieving the best therapeutic effect. The sensor unit 20 preferably also provides the user with feedback as to whether he has achieved his target oscillation frequency. This may be done in various different ways, such as by displaying a legend on the display: "Flow OK", "Flow Too High" or "Flow Too Low". Alternatively, the screen 24, or a part of the screen, could change colour to indicate whether flow was too high or low, or a sound signal could be produced.

As mentioned above, the setting of the dial 5 on the therapy device 100 affects the frequency and resistance to flow through the device. This is set by the user to achieve the maximum beneficial effect. The sensor unit 20 could be arranged to compute a measure of the flow rate and pressure generated from the measured frequency and from knowledge of the setting of the dial 5, as entered into the sensor unit by the user or clinician.

The acoustic sensor unit 20 can be highly sensitive to the sound produced by the therapy device 100 since this is in a relatively small range of frequencies. By filtering out other frequencies it is possible to use high gain amplification for maximum sensitivity.

The sensor unit 20 described above is a dedicated unit separate from the therapy device 100. The sensor unit need not be dedicated to monitoring use of the therapy device but could instead be a multifunction unit. In this respect, the sensor unit could be provided as a program application in a general purpose computer, using the microphone built in the computer. More particularly, the program application could be supported within a mobile phone, or in a laptop or tablet computer. The program application could be arranged to stop automatically after the elapse of a predetermined time without sensing any sound of the characteristic frequencies.

It will be appreciated that there are many different ways in which information obtained by the sensor unit can be represented so that it is provided to the user and clinician in the most useful manner.

Apparatus of the present invention can be used with any conventional respiratory therapy apparatus that produces a sound signal. The therapy apparatus may be combined with other treatments such as nebulisation or the administration of aerosol medication.

The present invention enables someone using an existing, conventional therapy device to be provided with useful data about its use. In this way, the user can be made more aware of how well he is complying with the prescribed therapy programme and can modify his use of the device accordingly to achieve maximum benefit. The clinician is also able to check patient compliance so that he can identify whether any deterioration in a patient's condition is due to lack of compliance or if alternative treatment is needed.

## Claims

1. Respiratory therapy apparatus including a vibratory PEP therapy device (100) including a valve (11, 12) that is opened and closed by breathing through the device such as to produce an oscillating resistance to breathing through the device, the apparatus includes a sensor (20) responsive to acoustic pressure waves transmitted through air caused by movement of the valve, the sensor (20) is arranged to provide a signal indicative of use of the device, and the sensor (20) is separate from the device (100) but placed close to it to receive the acoustic pressure waves transmitted from the device (100) through air to the sensor (20), **characterised in that**
the valve (11, 12) includes a valve element (11) on a rocker arm (12) that opens and closes an opening (10) during exhalation through the apparatus,
the oscillating movement of the rocker arm (12) producing an audible sound being transmitted through the surrounding air as the acoustic pressure waves,
the sensor (20) is arranged to provide a signal indicative of the frequency of oscillation,
the sensor (20) is contained in a unit including a display (24) on which is represented the frequency of oscillation detected by the sensor (20).

2. Respiratory therapy apparatus according to Claim 1, wherein the sensor (20) is arranged to provide a signal indicative of one or more of the following: when the device (100) is used, the duration of use.

3. Respiratory therapy apparatus according to claim 1 or 2, wherein the unit is a mobile phone and the sensor is the microphone of the phone, and that the phone is programmed to respond to the sound made by the apparatus during use.

## Patentansprüche

1. Atemtherapievorrichtung mit einer Vibrations-PEP-Therapieeinrichtung (100) mit einem Ventil (11, 12), das durch Atmen durch die Einrichtung geöffnet und geschlossen wird, um einen Oszillationswiderstand gegen die Atmung durch die Einrichtung zu erzeugen, wobei die Vorrichtung einen Sensor (20) umfasst, der auf durch Luft übertragene Schalldruckwellen anspricht, die durch die Bewegung des Ventils verursacht werden, wobei der Sensor (20) dazu beschaffen ist, ein Signal, das auf die Verwendung der Einrichtung hinweist, bereitzustellen, und wobei der Sensor (20) von der Einrichtung (100) getrennt, aber nahe dieser angeordnet ist, um die Schalldruckwellen zu empfangen, die von der Einrichtung (100) durch Luft zum Sensor (20) übertragen werden, **dadurch gekennzeichnet, dass**
das Ventil (11, 12) ein Ventilelement (11) an einem Kipphebel (12) umfasst, der eine Öffnung (10) während des Ausatmens durch die Vorrichtung öffnet und schließt,
wobei die oszillierende Bewegung des Kipphebels (12) einen hörbaren Ton erzeugt, der durch die umgebende Luft als die Schalldruckwellen übertragen wird,
wobei der Sensor (20) dazu beschaffen ist, ein Signal bereitzustellen, das auf die Oszillationsfrequenz hinweist,
wobei der Sensor (20) in einer Einheit mit einer Anzeige (24) enthalten ist, auf der die durch den Sensor (20) detektierte Oszillationsfrequenz dargestellt wird.

2. Atemtherapievorrichtung nach Anspruch 1, wobei der Sensor (20) dazu beschaffen ist, ein Signal bereitzustellen, das auf eines oder mehrere der Folgenden hinweist: wann die Einrichtung (100) verwendet wird, die Dauer der Verwendung.

3. Atemtherapievorrichtung nach Anspruch 1 oder 2, wobei die Einheit ein Mobiltelefon ist und der Sensor das Mikrophon des Telefons ist, und das Telefon so programmiert ist, dass es auf den durch die Vorrichtung während der Verwendung erzeugten Ton anspricht.

## Revendications

1. Appareil de thérapie respiratoire comprenant un dispositif de thérapie PEP vibratoire (100) comprenant une valve (11, 12) qui est ouverte et fermée en respirant à travers le dispositif afin de produire une résistance oscillante à la respiration à travers le dispositif, l'appareil comprend un capteur (20) sensible aux ondes de pression acoustique transmises par l'air provoqué par le mouvement de la valve, le capteur (20) est agencé pour fournir un signal indicatif de l'utilisation du dispositif, et le capteur (20) est séparé du dispositif (100) mais placé à proximité de ce dernier pour recevoir les ondes de pression acoustique transmises par le dispositif (100), par le biais de l'air, au capteur (20), **caractérisé en ce que** :
la valve (11, 12) comprend un élément de valve (11) sur un culbuteur (12) qui ouvre et ferme une ouverture (10) pendant l'expiration à travers l'appareil,
le mouvement oscillant du culbuteur (12) produisant un son audible qui est transmis par l'air environnant sous forme d'ondes de pression acoustique,
le capteur (20) est agencé pour fournir un signal indicatif de la fréquence d'oscillation,
le capteur (20) est contenu dans une unité comprenant un affichage (24) sur lequel est représenté la fréquence d'oscillation détectée par le capteur (20).

2. Appareil de thérapie respiratoire selon la revendication 1, dans lequel le capteur (20) est agencé pour fournir un signal indicatif d'un ou de plusieurs éléments suivants : le moment où le dispositif (100) est utilisé, la durée d'utilisation.

3. Appareil de thérapie respiratoire selon la revendication 1 ou 2, dans lequel l'unité est un téléphone mobile et le capteur est le microphone du téléphone et en ce que le téléphone est programmé pour répondre au son fait par l'appareil pendant l'utilisation.
